# EUROPEAN PATENT APPLICATION

(11) **EP 3 943 585 A1**
(43) Date of publication of application: **26.01.2022**
(21) Application number: 19921125.1
(22) Date of filing: 09.10.2019
(51) Int. Cl.: C12M 1/00, F16L 53/30, F17D 1/05

(54) **ORGANIC COMPOUND PRODUCTION SYSTEM**

(30) Priority: 22.03.2019 JP 2019054450
(71) Applicant: SEKISUI CHEMICAL CO., LTD., Osaka-shi Osaka 530-8565 (JP)
(72) Inventor: HASEGAWA Tomoya, Tsukuba-shi, Ibaraki 300-4292 (JP); HAMACHI Kokoro, Tsukuba-shi, Ibaraki 300-4292 (JP)
(74) Representative: Ter Meer Steinmeister & Partner
(86) International application number: PCT/JP2019/039892
(87) International publication number: WO 2020/194808

(57) **Abstract**

The present invention provides a means to prevent the clogging of pipes by freezing of gas discharged from a fermenter, especially in a low temperature environment such as in winter. Disclosed is an organic compound production system (10A) for producing an organic compound by microbial fermentation, including: a catalytic reactor (1) comprising a reactor containing a biocatalyst for synthesizing an organic compound; a valve (3) for discharging exhaust gas withdrawn from the catalytic reactor (1); and a pipe (6A) connecting the catalytic reactor (1) and the valve (3), wherein at least a part of the pipe (6A) is covered with an insulator (4).

## Description

### TECHNICAL FIELD

The present invention relates to an organic compound production system.

Priority is claimed on Japanese Patent Application No. 2019-054450, filed March 22, 2019, the contents of which are incorporated herein by reference.

### BACKGROUND ART

In recent years, researches have been made for practical implementation of a method for producing an organic compound such as ethanol by microbial fermentation of a carbon monoxide-containing synthesis gas acquired from an exhaust gas from ironworks and the like (see, for example, Patent Document 1). The process of such production of an organic compound involves a synthetic gas injected into a fermenter and a gas discharged (a gas not used in the microbial fermentation and a gas produced by the microbial fermentation). Therefore, as a general practice for adjusting the internal pressure of the fermenter, the amount of gas discharged is controlled by a valve. Such a valve is connected to the fermenter via a pipe.

### Prior Art References

### Patent Document

Patent Document 1: International Patent Application Publication No. 2011/087380

### DISCLOSURE OF INVENTION

### Problems to be Solved by the Invention

However, it has been found that, due to factors such as the presence of water in the gas discharged from the fermenter and the tendency of the pipe being cooled, the pipe may be clogged by freezing, especially in a low temperature environment such as in winter. Means to Solve the Problems

The present inventors have made intensive studies in order to solve the above problems. As a result, they have found that the above problems can be solved by covering a pipe with an insulator, and have completed the present invention.

[1] An organic compound production system for producing an organic compound by microbial fermentation, including:
   a catalytic reactor comprising a reactor containing a biocatalyst for synthesizing an organic compound,
   a valve configured to control discharge of an exhaust gas withdrawn from the catalytic reactor, and
   a pipe connecting the catalytic reactor and the valve,
   wherein at least a part of the pipe is covered with an insulator.
[2] The organic compound production system according to claim 1, further including a heating means configured to heat the at least a part of the pipe, wherein the pipe and the heating means are covered with the insulator.
[3] The organic compound production system according to [1] or [2], wherein at least a joint between the valve and the pipe and a portion adjacent thereto are covered with the insulator.
[4] An organic compound production system for producing an organic compound by microbial fermentation, including:
   a catalytic reactor comprising a reactor containing a biocatalyst for synthesizing an organic compound,
   a temporary storage tank configured to temporarily store an exhaust gas withdrawn from the catalytic reactor,
   a valve configured to control discharge of the exhaust gas,
   a first pipe connecting the catalytic reactor and the temporary storage tank, and a second pipe connecting the temporary storage tank and the valve,
   wherein at least a part of the second pipe is covered with an insulator.
[5] The organic compound production system according to [4], further including a heating means configured to heat the at least a part of the second pipe, wherein the at least a part of the second pipe and the heating means are covered with the insulator.
[6] The organic compound production system according to [4] or [5], wherein at least a joint between the valve and the second pipe and a portion adjacent thereto are covered with the insulator.
[7] The organic compound production system according to any one of [4] to [6], wherein at least a part of the temporary storage tank is covered with the insulator.
[8] The organic compound production system according to [7], further including a heating means configured to heat the at least a part of the temporary storage tank, wherein the at least a part of the temporary storage tank and the heating means are covered with the insulator.
[9] The organic compound production system according to any one of [1] to [8], further including a third pipe provided downstream of the valve, wherein at least a part of the third pipe is covered with the insulator.
[10] The organic compound production system according to [9], further including a heating means configured to heat the at least a part of the third pipe, wherein the at least a part of the third pipe and the heating means are covered with the insulator.

### Effect of the Invention

The organic compound production system of the present invention can prevent freezing of pipes in a low temperature environment.

### BRIEF DESCRIPTION OF THE DRAWINGS

FIG. 1 is a schematic diagram of an organic compound production system according to the first embodiment of the present invention.
FIG. 2 is a schematic diagram of an organic compound production system according to the second embodiment of the present invention.

### DESCRIPTION OF THE EMBODIMENTS

Hereinbelow, the present invention will be described in detail with reference to preferred embodiments of the present invention. However, these embodiments are described for example purpose only. The present invention is in no way limited by these embodiments.

### <First Embodiment>

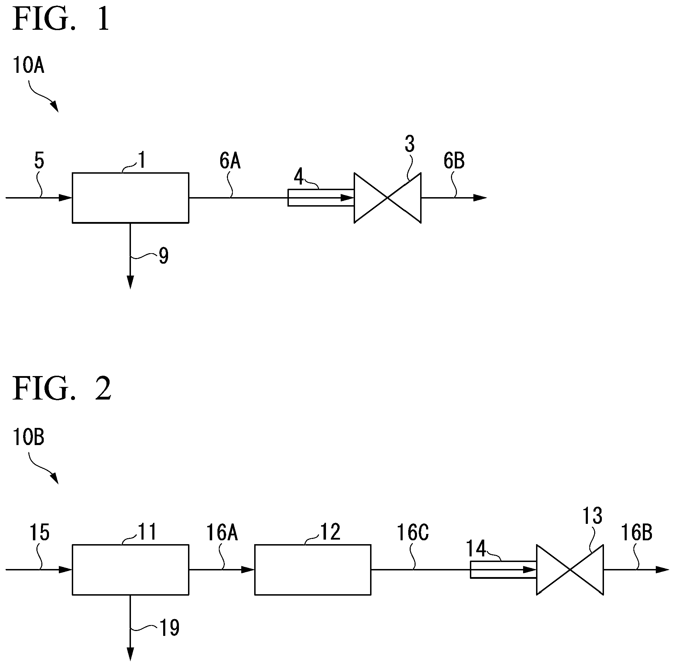

FIG. 1 is a schematic view of the organic compound production system according to the first embodiment of the present invention. The organic compound production system 10A shown in FIG. 1 includes a catalytic reactor 1 having a reactor containing a biocatalyst for synthesizing the organic compound, a valve 3 for controlling the discharge of exhaust gas withdrawn from the catalytic reactor, and a first pipe 6A connecting the catalytic reactor 1 and the valve 3. In this embodiment, at least a part of the first pipe 6A is covered with the insulator 4. The raw material gas supply pipe 5 supplies the organic compound raw material to the catalytic reactor 1. Further, the organic compound withdrawal pipe 9 withdraws the organic compound produced by the catalytic reactor 1. The third pipe 6B discharges the exhaust gas discharged from the valve to the outside of the system. The arrows in FIG. 1 indicate the directions of the gas flow in the pipes. That is, the organic compound production system 10A according to the first embodiment includes the raw material gas supply pipe 5, the catalytic reactor 1, the organic compound withdrawal pipe 9, the first pipe 6A, the insulator 4, the valve 3, and the third pipe 6B. More detailed description will be given below.

### [Raw material gas supply pipe 5]

The raw material gas supply pipe 5 supplies the raw material gas to the catalytic reactor 1.

The raw material gas is not particularly limited, and may be, for example, a synthetic gas containing carbon monoxide and hydrogen obtained by partial oxidation of a carbon source, or the like. In addition, the raw material gas may also include nitrogen, water vapor and the like, if necessary.

The carbon source is not particularly limited, and may be waste including plastics or resins, garbage, coke, or the like.

When the raw material gas contains impurities, it is preferable to use a purified raw material gas as the raw material gas.

### [Catalytic reactor 1]

The catalytic reactor 1 includes a reactor containing a biocatalyst for synthesizing an organic compound. This enables production of an organic compound by microbial fermentation.

Examples of the organic compound include, but are not particularly limited to, alcohols, organic acids, fatty acids, fats and oils, ketones, biomass, sugars and the like. More specific examples include ethanol, isopropyl alcohol, acetone, acetic acid, butanediol and the like. The use of the produced organic compound is not particularly limited, and the organic compound may be used as raw materials for resins such as plastics and rubbers, fuels, and the like.

The biocatalyst is not particularly limited. For example, when ethanol is produced as the organic compound from a synthetic gas (gas containing carbon monoxide and hydrogen), it is preferable to use Clostridium autoethanogenum, Clostridium ljungdahlii, Clostridium aceticum, Clostridium carboxidivorans, Moorella thermoacetica, Acetobacterium woodii, or the like.

The reactor preferably has a microbial fermenter, a thermostat for keeping the liquid medium in the microbial fermenter warm, and a stirring means for stirring the liquid medium in the microbial fermenter.

The temperature (culture temperature) of the liquid medium in the microbial fermenter is preferably about 30 to 45 °C, more preferably about 33 to 42 °C, and even more preferably about 36.5 to 37.5 °C.

The pressure in the microbial fermenter may be normal pressure, but is preferably about 10 to 300 kPa (gauge pressure), more preferably about 20 to 200 kPa (gauge pressure). By keeping the pressure in the microbial fermenter within the above range, it is possible to further increase the reactivity of gas-utilizing bacteria while suppressing an increase in equipment cost due to an excessive pressure load.

### [Organic compound withdrawal pipe 9]

The organic compound withdrawal pipe 9 has a function of withdrawing the reaction solution containing the organic compound synthesized in the catalytic reactor 1.

The withdrawn reaction solution is generally subjected to a purification step such as filtration or distillation to obtain a purified organic compound.

### [First pipe 6A]

The first pipe 6A connects the catalytic reactor 1 and the valve 3.

The material of the first pipe 6A is not particularly limited, and known materials can be used, the examples of which include a stainless steel, a copper alloy, a nickel alloy, titanium, copper, a copper alloy (such as brass, red brass, cupronickel, etc.), aluminum, an aluminum alloy, and a carbon steel pipe (such as SGP, STPY, STPG, STS, STPT, STPA, STPL, etc.). One of these materials may be used alone, or two or more of these may be used in combination.

Since the gas immediately after being discharged from the catalyst reactor 1 is allowed to flow through the first pipe 6A, the temperature of the exhaust gas is close to the culture temperature and hence can be regarded as a high temperature. However, the pipe is easily cooled in a low temperature environment, and when the exhaust gas is allowed to pass through the cooled first pipe for a long time, the temperature of the exhaust gas gradually decreases, which may result in freezing. Therefore, in the case where the first pipe 6A is long, the effect of the present invention can be exhibited more remarkably.

The first pipe 6A may have a bent portion. When the first pipe 6A has a bent portion, the bending angle is preferably more than 0° and 120° or less, more preferably more than 0° and 90° or less, and even more preferably more than 5° and 90° or less. When the first pipe 6A has a bent portion, the flow of exhaust gas inside the first pipe 6A changes, so that the first pipe 6A becomes more likely to be influenced by temperature change, and the cooling of the bent portion is more likely to cause the first pipe A to be frozen at that section. Therefore, when the first pipe 6A has a bent portion, the effect of the present invention can be exhibited more remarkably.

That is, in actual installation of the organic compound production system, it is often inevitable to make the first pipe 6A long and bent, and it is not easy to change the design once the production system is installed. However, the present invention can surely prevent freezing of the first pipe 6A even in such a case.

### [Insulator 4]

The insulator 4 covers at least a part of the first pipe. This enables prevention or suppression of the cooling of the first pipe described above, whereby the clogging of the first pipe due to freezing can be prevented or suppressed.

Examples of the material of the insulator 4 include calcium silicate, rock wool, glass wool, polyethylene foam, urethane foam, and polystyrene foam. One of these materials may be used alone, or two or more of these may be used in combination. When two or more types of the materials are used in combination, it is possible to use a multilayered insulator having multiple layers of difference materials, in which, for example, the inside (pipe side) of the insulator 14 is formed of glass wool, while the outside is formed of a heat-resistant polyethylene foam (having a heat-resistant temperature of 100 to 120 °C).

The shape of the insulator is not particularly limited, but is preferably a tubular shape from the viewpoint of efficient heat insulation.

The heat-resistant temperature of the insulator is preferably 100 °C or higher, more preferably 120 °C or higher, and even more preferably 150 °C or higher, for providing a heating means described later, preventing deterioration, and the like. The heat-resistant temperature can be measured according to JIS K7226.

The portion to be covered by the insulator is not particularly limited, but is preferably a portion distant from the joint with the catalyst reactor 1, and more preferably a portion in the vicinity of the joint with the valve 3. At a portion distant from the joint with the catalytic reactor 1, the exhaust gas is gradually cooled so that freezing is likely to occur. Therefore, it is preferable to keep this portion warm. Further, particularly in the vicinity of the joint with the valve 3, the flow path is often narrowed where the freezing is more likely to occur, so that it is preferable to keep this portion warm. That is, in one embodiment of the present invention, it is preferable that at least the valve joint of the pipe and the portion adjacent thereto are covered with an insulator.

The insulator 4 may cover a part other than the first pipe. For example, the insulator 4 may cover at least a part of at least one member selected from the group consisting of the catalytic reactor 1, the organic compound withdrawal pipe 9, the valve 3, and the third pipe 6B. Of these, it is preferable to cover at least a part of either one or both of the valve 3 and the third pipe 6B.

When the first pipe 6Ain the vicinity of the joint with the valve 3 is covered with the insulator, the coverage with the insulator is preferably 10 % or more, more preferably 20 % or more, even more preferably 30 % or more, of the total length of the first pipe 6A, as measured from the joint with the valve 3. From the viewpoint of cost, the coverage with the insulator is preferably 90 % or less, and more preferably 80 % or less.

When the insulator 4 covers only a part of the first pipe 6A, the insulator 4 may continuously cover the first pipe 6A, or may discontinuously cover the first pipe 6A.

### [Valve 3]

The valve 3 controls discharge of the exhaust gas withdrawn from the catalytic reactor.

The valve is not particularly limited, and a known valve can be appropriately adopted. The valve may be controlled manually or automatically. When the valve is controlled automatically, the composition of the exhaust gas, the amount of gas, and the like may be monitored, and the valve may be controlled by the control unit based on the acquired monitoring information.

### [Third pipe 6B]

Through the third pipe 6B, the gas from the valve 3 is discharged to the outside of the system. The discharged gas can be appropriately applied to incineration, reuse for culture, and the like. The material and the like of the third pipe 6B may be the same as those of the first pipe 6A.

### [Heating means]

In one embodiment, a heating means may be further provided.

The heating method of heating means may be direct heating or indirect heating.

The direct heating is not particularly limited, and examples thereof include those using an electric heater or the like.

Examples of the indirect heating method include those using a heating medium such as water and an antifreeze. In this context, the heat source for heating the heating medium may be, for example, an electric heater, heat generated in the organic compound production system, sunlight, or the like.

Of these, the heating method of the heating means is preferably indirect heating, preferably heating using water as a heating medium, and more preferably heating by steam. In this context, the heat source for the indirect heating is preferably heat generated in the organic compound production system or sunlight, and more preferably heat generated in the organic compound production system. Therefore, according to one preferred embodiment, the heating means is preferably steam heated by the heat generated in the organic compound production system.

As the application form of the heating means, a known method can be appropriately adopted depending on the heating method of the heating means. For example, in the case of heating by steam, heating by a steam pipe is preferable. For heating by a steam pipe, the steam pipe may be disposed so as to extend parallel to and in contact with the heating target (preferably the pipe as described later), or may be spirally wound around the heating target. Of these, it is preferable to spirally wind the steam pipe around the heating target in that efficient heating with one pipe is possible.

The application location of the heating means is not particularly limited, and examples thereof include the pipe, the valve, the catalytic reactor, the raw material gas supply pipe, the organic compound withdrawal pipe, and the insulator. Of these, it is preferable to heat the pipe, the valve, the catalytic reactor, and the insulator, it is more preferable to heat the pipe and the valve, and it is even more preferable to heat the pipe. When heating the pipe, it is preferable to heat its portion covered with the insulator from the viewpoint of high efficiency. That is, in one preferred embodiment, it is preferable that at least a part of the pipe is heated by the heating means, and the pipe and the heating means are covered with the insulator. Further, in a more preferable embodiment, it is preferable that at least a part of the pipe is heated by contact with the steam pipe, and the heating means and the steam pipe are covered with the insulator. The heating means may be applied to one location or two or more locations.

The heating means may be controlled by the control unit. The control unit starts the heating when it determines that heating is necessary, and stops the heating when it determines that heating is unnecessary. The determination on whether or not the heating is necessary is preferably made utilizing the information acquired by a sensor. For example, when the pipe is heated, a sensor for measuring the flow rate of exhaust gas inside the pipe is installed. When the flow rate of exhaust gas falls below a predetermined value, the heating is started by the control unit, so as to prevent clogging of the pipe.

### <Preferred example of the first embodiment>

In the first embodiment, it is preferable that at least a part of at least one member selected from the group consisting of the first pipe 6A, the valve 3 and the third pipe 6B is covered with an insulator, and it is more preferable that at least one member selected from the group consisting of the first pipe 6A in the vicinity of the joint with the valve 3, the valve 3, and the third pipe 6B in the vicinity of the joint with the valve 3 is covered with an insulator. In this context, the "first pipe 6A in the vicinity of the joint with the valve 3" means a region of the first pipe 6A which extends from the joint with the valve 3 and is within 30 %, preferably 20 %, more preferably 10 %, of the total length of the first pipe 6A. Similarly, the "third pipe 6B in the vicinity of the joint with the valve 3" means a region of the third pipe 6B which extends from the joint with the valve 3 and is within 30 %, preferably 20 %, more preferably 10 %, of the total length of the third pipe 6B. Further, it is preferable that the pipes are heated by contact with a steam pipe, and that the pipes and the steam pipe are covered with the insulator.

### <Second Embodiment>

FIG. 2 is a schematic diagram of an organic compound production system according to the second embodiment of the present invention. The organic compound production system 10B shown in FIG. 2 includes: a catalytic reactor 11 having a reactor containing a biocatalyst for synthesizing an organic compound; a temporary storage tank 12 for temporarily storing the exhaust gas withdrawn from the catalytic reactor 11; a valve 13 for controlling the discharge of the exhaust gas; a first pipe 16A connecting the catalytic reactor and the temporary storage tank; and a second pipe 16C connecting the temporary storage tank and the valve. In this production system, at least a part of the second pipe is covered with the insulator 14. Similarly to the first embodiment, the organic compound production system 10B includes a raw material gas supply pipe 15, an organic compound withdrawal pipe 19, and a third pipe 16B.

The organic compound production system 10B according to the second embodiment has a temporary storage tank. The presence of the temporary storage tank 12 in the system enables easier adjustment of the internal pressure of the catalytic reactor including the fermenter and the like. However, the exhaust gas stored in the temporary storage tank 12 is caused to stay in the temporary storage tank for a certain period of time, and the temperature of the exhaust gas usually gradually decreases in the temporary storage tank. Further, when the exhaust gas is transported to the valve 13 through the second pipe 16C with the exhaust gas temperature being cooled to a low temperature, freezing may be more likely to occur if the second pipe 16C is in a low temperature environment. Therefore, in the present embodiment, at least a part of the second pipe is covered with the insulator.

Hereinbelow, the second embodiment of the present invention will be described in more detail, but the raw material gas supply pipe, the catalytic reactor, and the organic compound withdrawal pipe are the same as those in the first embodiment, and hence the descriptions thereof will be omitted.

### [First pipe 16A]

The first pipe 16A connects the catalytic reactor and the temporary storage tank. The material and the like therefor may be the same as those in the first embodiment.

### [Temporary storage tank 12]

The temporary storage tank 12 is configured to temporarily store the exhaust gas withdrawn from the catalytic reactor.

The temporary storage tank 12 is not particularly limited, and examples thereof include a general knockout container for performing gas-liquid separation.

### [Second pipe 16C]

The second pipe 16C connects the temporary storage tank and the valve. As described above, since the exhaust gas flowing through the second pipe 16C is stored in the temporary storage tank for a predetermined period of time, the gas temperature there is lower than when the exhaust gas is discharged from the catalytic reactor including the fermenter or the like. Therefore, at least a part of the second pipe 16C is kept warm with an insulator described later.

The material and the like of the second pipe 16C may be the same as those of the first pipe 6A.

The dimensions and materials of the first pipe 16A and the second pipe 16C are not particularly limited, and known ones can be used as appropriate. Generally, the second pipe 16C is designed to be longer than the first pipe 16A. For example, the length of the second pipe 16C is usually 1 to 10 times, preferably 1 to 5 times, and more preferably 1 to 3 times the length of the first pipe 16A.

Further, as in the first embodiment, the effect of the present invention can be exhibited more remarkably when either one or both of the first pipe 16A and the second pipe 16C are long and/or have a bent portion.

### [Insulator 14]

The insulator 14 covers at least a part of the second pipe. This enables prevention or suppression of the cooling of the second pipe described above, whereby the clogging of the second pipe due to freezing can be prevented or suppressed.

The material and the like of the insulator 14 may be the same as those in the first embodiment.

Further, the insulator may cover a part other than the second pipe as in the first embodiment. For example, the insulator may cover at least a part of at least one member selected from the group consisting of the catalytic reactor 11, the organic compound withdrawal pipe 19, the first pipe 16A, the temporary storage tank 12, the valve 13, and the third pipe 16B. Of these, it is preferable to cover at least a part of at least one member selected from the group consisting of the first pipe 16A, the temporary storage tank 12, the valve 13, and the third pipe 16B; it is more preferable to cover at least a part of at least one member selected from the group consisting of the temporary storage tank 12, the valve 13, and the third pipe 16B; and it is even more preferable to cover at least a part of one or both of the valve 13 and the third pipe 16B. In one preferred embodiment, at least a part of the temporary storage tank is covered with the insulator.

### [Valve 13]

The valve 13 controls the discharge of the exhaust gas. The type, control, etc. of the valve may be the same as those in the first embodiment.

### [Third pipe 16B]

The third pipe 16B is provided on the downstream side of the valve. The discharged gas can be appropriately applied to incineration, reuse for culture, and the like. The material and the like of the third pipe 16B may be the same as those of the second pipe 16A.

### [Heating means]

In one embodiment, a heating means may be further provided. The heating means is as described above.

Also in the second embodiment, it is preferable that the steam pipe is spirally wound around the pipe.

The application location of the heating means is not particularly limited, and examples thereof include the pipe, the temporary storage tank, the valve, the catalytic reactor, the raw material gas supply pipe, the organic compound withdrawal pipe, and the insulator. Of these, it is preferable to heat the pipe, the temporary storage tank, the valve, the catalytic reactor, and the insulator; it is more preferable to heat the pipe, the temporary storage tank, and the valve; it is even more preferable to heat the valve and the pipe; and it is particularly preferable to heat the pipe. When heating the pipe, it is preferable to heat its portion covered with the insulator from the viewpoint of high efficiency. That is, in a preferable embodiment, it is preferable that at least a part of the pipe is heated by contact with the steam pipe, and the pipe and the steam pipe are covered with the insulator. The heating means may be applied to one location or two or more locations.

### <Preferred example of the second embodiment>

In the second embodiment, it is preferable that at least a part of at least one member selected from the group consisting of the second pipe 16C, the valve 13 and the third pipe 16B is covered with an insulator, and it is more preferable that at least one member selected from the group consisting of the second pipe 16C in the vicinity of the joint with the valve 13, the valve 13, and the third pipe 16B in the vicinity of the joint with the valve 13 is covered with an insulator. In this context, the "second pipe 16C in the vicinity of the joint with the valve 13" means a region of the second pipe 16C which extends from the joint with the valve 13 and is within 30 %, preferably 20 %, more preferably 10 %, of the total length of the second pipe 16C. Similarly, the "third pipe 16B in the vicinity of the joint with the valve 13" means a region of the third pipe 16B which extends from the joint with the valve 13 and is within 30 %, preferably 20 %, more preferably 10 %, of the total length of the third pipe 16B. Further, it is preferable that the pipes are heated by contact with a steam pipe, and that the pipes and the steam pipe are covered with the insulator.

### DESCRIPTION OF THE REFERENCE SIGNS

- 10A, 10B: Organic compound production system
- 1,11: Catalytic reactor
- 12: Temporary storage tank
- 3,13: Valve
- 4,14: Insulator
- 5,15: Raw material gas supply pipe
- 6A,16A: First pipe
- 6B,16B: Third pipe
- 16C: Second pipe
- 9,19: Organic compound withdrawal pipe

## Claims

1. An organic compound production system for producing an organic compound by microbial fermentation, comprising:
a catalytic reactor comprising a reactor containing a biocatalyst for synthesizing an organic compound,
a valve configured to discharge an exhaust gas withdrawn from the catalytic reactor, and
a pipe connecting the catalytic reactor and the valve,
wherein at least a part of the pipe is covered with an insulator.

2. The organic compound production system according to claim 1, further comprising a heating means configured to heat the at least a part of the pipe, wherein the at least a part of the pipe and the heating means are covered with the insulator.

3. The organic compound production system according to claim 1 or 2, wherein at least a joint between the valve and the pipe and a portion adjacent thereto are covered with the insulator.

4. An organic compound production system for producing an organic compound by microbial fermentation, comprising:
a catalytic reactor comprising a reactor containing a biocatalyst for synthesizing an organic compound,
a temporary storage tank configured to temporarily store an exhaust gas withdrawn from the catalytic reactor,
a valve configured to control discharge of the exhaust gas,
a first pipe connecting the catalytic reactor and the temporary storage tank, and
a second pipe connecting the temporary storage tank and the valve,
wherein at least a part of the second pipe is covered with an insulator.

5. The organic compound production system according to claim 4, further comprising a heating means configured to heat the at least a part of the second pipe, wherein the at least a part of the second pipe and the heating means are covered with the insulator.

6. The organic compound production system according to claim 4 or 5, wherein at least a joint between the valve and the second pipe and a portion adjacent thereto are covered with the insulator.

7. The organic compound production system according to any one of claims 4 to 6, wherein at least a part of the temporary storage tank is covered with the insulator.

8. The organic compound production system according to claim 7, further comprising a heating means configured to heat the at least a part of the temporary storage tank, wherein the at least a part of the temporary storage tank and the heating means are covered with the insulator.

9. The organic compound production system according to any one of claims 1 to 8, further comprising a third pipe provided downstream of the valve, wherein at least a part of the third pipe is covered with an insulator.

10. The organic compound production system according to claim 9, further comprising a heating means configured to heat the at least a part of the third pipe, wherein the at least a part of the third pipe and the heating means are covered with the insulator.
